(19) Europäisches Patentamt
European Patent Office
Office européen des brevets

(11) Veröffentlichungsnummer: **0 071 839**
**B1**

(12) # EUROPÄISCHE PATENTSCHRIFT

(45) Veröffentlichungstag der Patentschrift: **25.09.85**

(51) Int. Cl.⁴: **C 07 C 143/78,** C 08 G 18/77

(21) Anmeldenummer: **82106664.4**

(22) Anmeldetag: **23.07.82**

(54) **Neue N,N-disubstituierte Sulfonamidgruppen aufweisende aromatische Diisocyanate, Verfahren zu ihrer Herstellung sowie ihre Verwendung als Aufbaukomponente bei der Herstellung von Polyurethanen.**

(30) Priorität: **04.08.81 DE 3130844**

(43) Veröffentlichungstag der Anmeldung: **16.02.83 Patentblatt 83/7**

(45) Bekanntmachung des Hinweises auf die Patenterteilung: **25.09.85 Patentblatt 85/39**

(84) Benannte Vertragsstaaten: **BE DE FR GB IT**

(56) Entgegenhaltungen: **EP-A-0 029 161**

(73) Patentinhaber: **BAYER AG, Konzernverwaltung RP Patentabteilung, D-5090 Leverkusen 1 Bayerwerk (DE)**

(72) Erfinder: **Kopp, Richard, Dr., Wolfskaul 12, D-5000 Köln 80 (DE)**
Erfinder: **Grögler, Gerhard, Dr., von-Diergardt-Strasse 46, D-5090 Leverkusen (DE)**
Erfinder: **Reiff, Helmut, Dr., Walter-Flex-Strasse 16, D-5090 Leverkusen (DE)**
Erfinder: **Dieterich, Dieter, Dr., Ludwig-Girtler-Strasse 1, D-5090 Leverkusen 1 (DE)**

EP 0 071 839 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue, N,N-disubstituierte Sulfonamidgruppen aufweisende aromatische Diisocyanate, mehrere Verfahren zu ihrer Herstellung durch Phosgenierung der entsprechenden Diamine, durch Abspaltung von tertiären Alkoholen aus den ihnen entsprechenden Bisurethanen tertiärer Alkohole mittels Phosgen oder durch Abspaltung von sekundären Aminen aus den ihnen entsprechenden, N,N-disubstituierten Bisharnstoffen mittels Chlorwasserstoff, sowie die Verwendung der neuen Diisocyanate als Aufbaukomponente bei der Herstellung von Polyurethankunststoffen.

Aromatische Monoisocyanate, deren aromatische Kerne durch N,N-Dialkylsulfonamidgruppen substituiert sind, sind bekannt (s. z.B. CH-PS 411 449; BE-PS 734 235; US-PS 3 576 872; NL-OS 7 105 350; JA-OS 7 316 502).

Sie werden z.B. als Kupplungsreagentien in der Farbfotografie, als Zwischenprodukte für Harze, Polymere, Farbstoffe, Bleichmittel, Pharmazeutica, Desinfektionsmittel oder Insektizide eingesetzt.

Bislang jedoch noch nicht bekannt geworden sind am aromatischen Kern N,N-disubstituierte Sulfonamidgruppen aufweisende aromatische Diisocyanate. In der FR-PS 1 465 165 wird lediglich die Herstellung von aromatischen Aminen aus Isocyanaten durch säurekatalysierte Thermolyse der entsprechenden s- oder t-Alkylcarbaminsäureester beschrieben. Hierbei werden u.a. auch N-Alkylsulfonamido-aryl(di)isocyanate als Ausgangsverbindungen für die Herstellung der Alkylcarbaminsäureester erwähnt. Es wird aber in keinem Beispiel auf diese Verbindungen eingegangen. Deshalb ist es sehr fraglich, ob diese Verbindungen in Substanz vorgelegen haben und eingesetzt worden sind. Wenn die entsprechenden N-Alkylsulfonamidoaryl(di)amine zur Herstellung der Isocyanate phosgeniert werden, besteht nämlich leicht die Gefahr, dass die N-Alkylsulfonamidgruppe in einer Nebenreaktion nach folgender Gleichung reagiert:

$$R\text{-}SO_2\text{-}NH\text{-}R + COCl_2 \rightarrow R\text{-}SO_2\text{-}\underset{|}{\overset{R}{N}}\text{-}CO\text{-}Cl + HCl$$

Durch die vorliegende Erfindung werden somit erstmals am aromatischen Kern N,N-disubstituierte Sulfonamidgruppen aufweisende aromatische Diisocyanate zur Verfügung gestellt. Bei diesen erfindungsgemässen Verbindungen handelt es sich um besonders interessante Ausgangsmaterialien für die Herstellung von Polyurethankunststoffen nach dem Isocyanat-Polyadditionsverfahren, da

1. die Diisocyanate bzw. die aus ihnen hergestellten Polyurethane physiologisch weitgehend unbedenkliche Substanzen darstellen dürften, weil die ihnen zugrundeliegenden N,N-disubstituierte Sulfonamidgruppen aufweisenden, bei der Hydrolyse der Diisocyanate bzw. der Polyurethane entstehehenden aromatischen Diamine nach dem derzeitigen Erkenntnisstand keinerlei Aktivität im Ames-Test* zeigen, wie am Beispiel von 1-(N,N-di-n-butyl-sulfonamido)-3,5-diamino-4-methylbenzol gefunden wurde;
2. die Reaktivität der Isocyanatgruppen der erfindungsgemässen Diisocyanate durch den induktiven Effekt der disubstituierten Sulfonamidgruppe stark erhöht wird, so dass die neuen Diisocyanate für Verbindungen mit gegenüber Isocyanatgruppen reaktionsfähigen Wasserstoffatomen besonders schnell reagierende Reaktionspartner darstellen;
3. durch den erhöhten Gehalt an Sauerstoff, Schwefel und Stickstoff der neuen Diisocyanate im Vergleich zu den klassischen Polyisocyanaten der Polyurethanchemie das Brandverhalten der mit den Diisocyanaten hergestellten Polyurethankunststoffe verbessert wird, und
4. die mit den neuen Diisocyanaten hergestellten Polyurethane verbesserte mechanische Eigenschaften insbesondere bei höheren Temperaturen aufweisen.

Gegenstand der Erfindung sind somit Diisocyanate der allgemeinen Formel

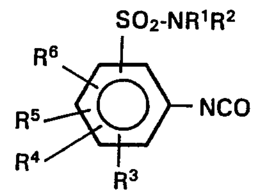

in der

$R^1$ und $R^2$ für gleiche oder verschiedene Reste stehen und Alkylreste mit 1 bis 18 Kohlenstoffatomen, Arylreste mit 6 bis 10 Kohlenstoffatomen, Cycloalkylreste mit 3 bis 7 Kohlenstoffatomen, Aralkylreste mit 7 bis 15 Kohlenstoffatomen oder Alkarylreste mit 7 bis 15 Kohlenstoffatomen bedeuten, oder zusammen mit dem Stickstoffatom einen gegebenenfalls weitere Heteroatome enthaltenden heterocyclischen Ring mit 3 bis 10 Ringgliedern bilden,

$R^3$, $R^4$ und $R^5$ für gleiche oder verschiedene Reste stehen und Wasserstoff, Alkylreste mit 1 bis 6 Kohlenstoffatomen, Arylreste mit 6 bis 10 Kohlenstoffatomen, Cycloalkylreste mit 3 bis 7 Kohlenstoffatomen, Aralkylreste mit 7 bis 15 Kohlenstoffatomen, Alkarylreste mit 7 bis 15 Kohlenstoffatomen oder Halogenatome bedeuten

$R^6$ für eine Isocyanatgruppe oder einen Rest der Formel

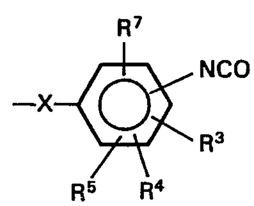

steht, wobei

$R^7$ für Wasserstoff oder einen Rest der Formel

$$-SO_2\ NR^1R^2$$

steht und

X für eine Methylen- oder eine Dimethylmethylen-Gruppe steht.

* vgl. z.B. Ames et Al., Proc. nat. Acad. Sci. (USA), 70, 2281-2285 (1973)

Gegenstand der Erfindung ist auch ein Verfahren zur Herstellung der neuen Diisocyanate, welches dadurch gekennzeichnet ist, dass man die den Diisocyanaten zugrundeliegenden Diamine in an sich bekannter Weise phosgeniert (1. erfindungsgemässes Verfahren).

Gegenstand der Erfindung ist auch ein Verfahren zur Herstellung der neuen Diisocyanate, welches dadurch gekennzeichnet ist, dass man die den Diisocyanaten entsprechenden Bisurethane tertiärer Alkohole mit Phosgen behandelt (2. erfindungsgemässes Verfahren).

Gegenstand der Erfindung ist auch ein Verfahren zur Herstellung der neuen Diisocyanate, welches dadurch gekennzeichnet ist, dass man den Diisocyanaten entsprechende, 2 Struktureinheiten der Formel

$$-NH-\overset{O}{\overset{\|}{C}}-NR^1R^2$$

aufweisende Bisharnstoffe unter Abspaltung von sekundären Aminen der Formel

$$HNR^1R^2$$

und gleichzeitiger Bildung der entsprechenden Ammoniumsalze der Formel

$$[H_2NR^1R^2]^+ \; Cl^{(-)}$$

mit Chlorwasserstoff behandelt (3. erfindungsgemässes Verfahren).

Gegenstand der Erfindung ist auch die Verwendung der neuen Diisocyanate als Aufbaukomponente bei der Herstellung von Polyurethankunststoffen nach dem Isocyanat-Polyadditionsverfahren.

Die erfindungsgemässen Diisocyanate entsprechen der o.g. allgemeinen Formel mit der o.g. Bedeutung der Reste $R^1$, $R^2$, $R^3$, $R^4$, $R^5$ und $R^6$. Bevorzugte erfindungsgemässe Diisocyanate sind solche der genannten Formel für welche

$R^1$ für einen Alkylrest mit 1 bis 6 Kohlenstoffatomen steht,

$R^2$ für einen Alkylrest mit 1 bis 18 Kohlenstoffatomen steht,

$R^3$ und $R^4$ für Wasserstoff stehen,

$R^5$ für Wasserstoff, einen Alkylrest mit 1 bis 4 Kohlenstoffatomen oder Chlor steht und

$R^6$ für eine Isocyanatgruppe steht.

Die neuen Verbindungen können nach den genannten, unterschiedlichen Verfahren hergestellt werden.

Bei der Herstellung der Diisocyanate durch Phosgenierung der ihnen zugrundeliegenden Diamine werden als Ausgangsmaterial Diamine der Formel

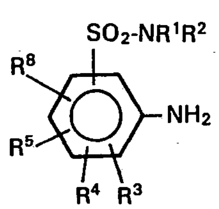

eingesetzt, wobei in dieser Formel

$R^1$, $R^2$, $R^3$, $R^4$ und $R^5$ die bereits genannte Bedeutung bzw. die bereits genannte bevorzugte Bedeutung haben,

$R^7$ und X die bereits genannte Bedeutung haben, und

$R^8$ für einen Rest der Formel

$-NH_2$ oder -X- 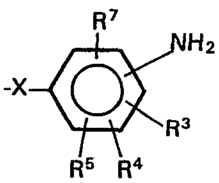

insbesondere für eine primäre Aminogruppe steht.

Die Herstellung derartiger Diamine ist beispielsweise in der deutschen Patentanmeldung P 3 012 800.8 entsprechend der europäischen Patentanmeldung 81 102 091.6 bzw. der US-Patentanmeldung 243 104 vom 12.3.1981 beschrieben. Sie beruht im wesentlichen darauf, Alkalisalze der entsprechenden aromatischen Dinitrosulfonsäuren nach bekannten Methoden in die entsprechenden Sulfochloride zu überführen, diese anschliessend durch Umsetzung mit sekundären Aminen $HNR^1R^2$ in Gegenwart einer Base in aromatische Dinitrosulfonamide zu überführen, um anschliessend diese zum Diamin zu hydrieren.

Auf diese Weise sind beispielsweise folgende Diamine zugänglich , die nach dem erstgenannten erfindungsgemässen Verfahren durch Phosgenierung in erfindungsgemässe Diisocyanate überführt werden können: 1-(N,N-di-n-butyl-sulfonamido)-3,5-diamino-4-methyl-benzol, 1-(N-Ethyl-N-phenyl-sulfonamido)-2,4-diamino-benzol, 1-(N,N-di-n-butyl-sulfonamido)-3-methyl-4,6-diamino-benzol, 1-(N-Methyl-N-stearyl-sulfonamido)-3,5-diamino-4-methyl-benzol oder 1-[(2,5-Diamino-4-methylphenyl)-sulfonyl]-pyrrolidin.

Ein weiterer Weg zu Diaminen die durch eine einfache Phosgenierungsreaktion in erfindungsgemässe Diisocyanate überführt werden können, besteht beispielsweise darin, dass man ein aromatisches Diamin, beispielsweise 4,4'-Diaminodiphenylmethan, mit Essigsäureanhydrid an den Stickstoffatomen acyliert, anschliessend beide aromatische Kerne sulfochloriert, das Sulfochlorierungsprodukt durch Umsetzung mit einem sekundären Diamin in das Bissulfonamid überführt und schliesslich sauer oder alkalisch zum Bis-sulfonamid-diamin hydrolysiert. Auf diese Weise sind beispielsweise Diamine wie 3,3'-Bis-(N,N'-dibutyl-sulfonamido)-4,4'-diamino-diphenylmethan zugänglich. Diamine dieses Typs sind z.B. in J. amer. chem. soc. 74, (1952) Seite 57-99 oder in US-PS 3 639 342 beschrieben.

Bei der Durchführung des 1. erfindungsgemässen Verfahrens erfolgt die Phosgenierung der Diamine nach den üblichen Methoden ein- oder zweistufig (Kalt-Heiss-Phosgenierung) in Gegenwart geeigneter Lösungsmittel wie 1,2-Dichlorethan, Chlorbenzol oder o-Dichlorbenzol im Temperaturbereich von ca. —20°C bis +200°C.

Ein weiteres Verfahren zur Herstellung der erfindungsgemässen Diisocyanate besteht in der Umsetzung der den Diisocyanaten entsprechenden Bisurethane tertiärer Alkohole mit Phosgen. Bei diesen Bisurethanen handelt es sich um Verbindungen der Formel

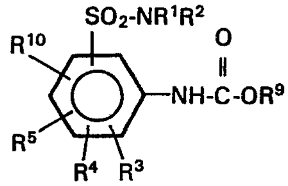

in welcher'

$R^1$, $R^2$, $R^3$, $R^4$ und $R^5$ die bereits genannte Bedeutung bzw. die bereits genannte bevorzugte Bedeutung haben,

$R^9$ für einen tertiären Alkylrest mit vorzugsweise 4 bis 8 Kohlenstoffatomen steht und

$R^{10}$ für einen Rest der Formel

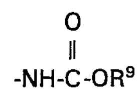

oder der Formel

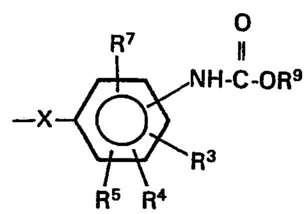

steht, wobei

$R^7$ und X die bereits genannte Bedeutung haben.

Diese Art der Herstellung der erfindungsgemässen Diisocyanate erfolgt in weitgehender Analogie zum Verfahren der DE-OS 2 637 114, mit dem Unterschied, dass erfindungsgemäss anstelle der in dieser Vorveröffentlichung eingesetzten Phenolate sekundäre Diamine der Formel $HNR^1R^2$ mit dem Sulfochloridgruppen umgesetzt werden.

Die bei diesem erfindungsgemässen Verfahren mit Phosgen zu behandelnden Bisurethane werden demzufolge hergestellt, indem aromatische, Sulfonamidgruppen-freie Diisocyanate in an sich bekannter Weise in die entsprechenden Isocyanato-Sulfochloride überführt (vgl. z.B. DE-OS 2 855 938), dann die Isocyanatgruppen mit einem tertiären Alkohol wie z.B. t-Butanol urethanisiert und anschliessend die Sulfochloridgruppen mit einem sekundären Amin zu Sulfonamidgruppen umgesetzt werden.

Diese letzte Stufe, die in der DE-OS 2 637 114 nicht vorbeschrieben ist, erfolgt vorzugsweise in Gegenwart einer alkalisch reagierenden Verbindung zur Neutralisation des sich bildenden Chlorwasserstoffs.

Als sekundäre Amine der Formel $HNR^1R^2$ für diese letzte Stufe eignen sich beispielsweise Dimethylamin, Diethylamin, Di-n-propylamin, Diisopropylamin, die isomeren, sekundären Butylamine oder Hexylamine, Morpholin, Pyrrolidin, N-Methylstarylamin, N-Methylanilin oder N-Ethylanilin.

Als alkalisch reagierende Verbindungen eignen sich z.B. Erdalkali- oder Alkalihydroxide oder auch überschüssige Mengen der vorgenannten sekundären Amine oder tertiäre Amine wie z.B. Pyridin oder Triethylamin.

Bei der Sulfonamidbildung wird das Mengenverhältnis der Reaktionspartner im allgemeinen so gewählt, dass ein Molverhältnis Sulfochloridgruppen : sekundäres Amin von 1 : 1 bis 1 : 2,5 vorliegt. Besonders bevorzugt setzt man die genannten Reaktionspartner in etwa äquimolaren Mengen ein. Der bei der Reaktion freiwerdende Chlorwasserstoff kann, wie schon erwähnt, entweder durch überschüssiges Amin $HNR^1R^2$ oder durch den Zusatz einer weiteren Base gebunden werden.

Die Umsetzung kann durch gleichzeitiges Zusammenmischen aller 3 Komponenten oder aber durch Vorlegen einer bzw. zweier Komponenten und anschliessende Zugabe der beiden übrigen bzw. der dritten Komponente vorgenommen werden. So kann man beispielsweise das Amin vorlegen und das Bisurethan-sulfochlorid und die alkalisch reagierende Verbindung gleichzeitig getrennt zulaufen lassen. Die besten Ausbeuten erhält man bei der Umsetzung etwa äquimolarer Mengen Sulfochlorid und Amin, wenn man das Sulfochlorid in einem unpolaren organischen Lösungsmittel wie z.B. Toluol suspendiert vorlegt und das Amin und die Base gleichzeitig zulaufen lässt.

Als Reaktionsmedium können jedoch auch andere inerte, d.h. polare organische Lösungsmittel wie z.B. Tetrahydrofuran, Dioxan, Diethylether oder Aceton verwendet werden. Die Sulfonamidbildung wird im allgemeinen innerhalb des Temperaturbereichs von —20°C bis 60°C durchgeführt. Besonders bevorzugt erfolgt die Sulfonamidbildung in einem Eintopfverfahren unmittelbar im Anschluss an die Urethanisierung der Isocyanatgruppen im bereits dort verwendeten Lösungsmittel ohne Reindarstellung der bei der Urethanisierung anfallenden Zwichenstufe.

Das zweite erfindungsgemässe Verfahren zur Herstellung der neuen Diisocyanate unter Behandlung der Sulfonamidgruppen aufweisenden Bisurethane mit Phosgen erfolgt in völliger Analogie zur Verfahrensweise der DE-OS 2 637 114. Dies bedeutet, dass man die Sulfonamidgruppen aufweisenden Bisurethane beispielsweise in einem geeigneten Lösungsmittel wie 1,2-Dichlorethan, Chlorbenzol oder o-Dichlorbenzol löst und die Lösung bei ca. 0°C bis 100°C mit gasförmigem Phosgen behandelt. Anschliessend wird überschüssiges Phosgen mit einem Inertgas wie z.B. Stickstoff ausgetrieben und das Lösungsmittel abgedampft. Das erfindungsgemässe Diisocyanat fällt hierbei als Rückstand an und kann gegebenenfalls destillativ oder durch Umkristallisieren gereinigt werden.

Die bei diesem erfindungsgemässen Verfahren einzusetzenden Bisurethane können im übrigen auch aus den entsprechenden Diaminoarylsulfonsäuren erhalten werden, indem man diese mit Phosgen in die entsprechenden Diisocyanatoarylsulfochloride überführt und diese wie beschrieben weiterverarbeitet.

Ein weiterer Weg zu den erfindungsgemässen Diisocyanaten besteht darin, die ihnen entsprechenden N,N-disubstituierten Harnstoffe unter Einwirkung von Chlorwasserstoff unter Abspaltung des dem Harnstoff entsprechenden sekundären Amins und dessen Überführung in das entsprechende Ammoniumchlorid zu spalten.

Die bei diesem Verfahren einzusetzenden Bisharnstoffe entsprechen der Formel

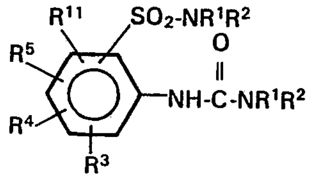

in welcher
die Reste $R^1$, $R^2$, $R^3$, $R^4$ und $R^5$ die bereits genannte Bedeutung bzw. die bereits genannte bevorzugte Bedeutung haben,
$R^{11}$ für einen Rest der Formel

$$-NH-CO-NR^1R^2$$

oder der Formel

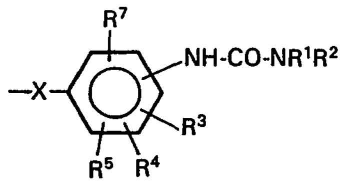

steht, wobei
$R^7$ und X die bereits genannte Bedeutung haben.

Derartige Bisharnstoffe entstehen beispielsweise durch Umsetzung der entsprechenden Diisocyanatoarylsulfochloride mit sekundären Aminen $HNR^1R^2$, wobei für jedes Mol an Isocyanatgruppen mindestens 1 Mol des sekundären Amins und für jede Sulfochloridgruppe mindestens 2 Mol des sekundären Amins zum Einsatz gelangt. Das 2. Mol an sekundärem Diamin bei der Umsetzung der Sulfochloridgruppen mit dem sekundären Amin dient hierbei, wie bereits oben beschrieben, zur Neutralisation des abzuspaltenden Chlorwasserstoffs.

Die Umsetzung der Diisocyanato-aryl-sulfochloride mit den sekundären Diaminen verläuft spontan in praktisch quantitativer Ausbeute. Die Umsetzung wird vorzugsweise in polaren oder unpolaren Lösungsmitteln der bereits beispielhaft genannten Art innerhalb des Temperaturbereichs von 20-80°C durchgeführt.

Die dabei anfallenden Lösungen der Sulfonamidgruppen aufweisenden Bisharnstoffe können dann sofort dem erfindungsgemässen Verfahren, d.h. der Behandlung mit Chlorwasserstoff, zugeführt werden. Selbstverständlich ist es jedoch auch möglich, die Bisharnstoffe zu isolieren und in einem anderen Lösungsmittel zu lösen. Das erfindungsgemässe Verfahren erfolgt im allgemeinen im Temperaturbereich von 100 bis 200°C unter inniger Durchmischung des zu spaltenden Bisharnstoffs mit vorzugsweise gasförmigem Chlorwasserstoff. Der Chlorwasserstoff wird vorzugsweise im molaren Überschuss eingesetzt. Besonders bevorzugt wird gasförmiger Chlorwasserstoff, gegebenenfalls zusammen mit einem Inertgas wie Stickstoff oder Kohlendioxid, innerhalb des genannten Temperaturbereichs in die vorgelegte Lösung des Bisharnstoffs eingeleitet. Im allgemeinen werden bei der Durchführung dieses erfindungsgemässen Verfahrens ca. 5- bis 50gew.-%ige Lösungen des Bisharnstoffs eingesetzt. Besonders bevorzugt sind solche Lösungsmittel, die unter den angegebenen Temperaturbedingungen noch nicht sieden. Besonders gut geeignet sind Xylol, Toluol, Monochlorbenzol, Dichlorbenzol oder Trichlorbenzol. Das nicht bildende Hydrochlorid des abgespaltenen sekundären Amins fällt in diesen Lösungsmitteln aus und kann beispielsweise durch Filtration oder durch Zentrifugieren entfernt werden.

Die danach vorliegende Lösung des erfindungsgemässen Diisocyanats wird, wie bereits oben ausgeführt, aufgearbeitet.

Die neuen Diisocyanate können nach allen bekannten Methoden der Polyurethanchemie anstelle der bisher üblichen Diisocyanate zur Herstellung von Polyurethankunststoffen verwendet werden.

In den nachfolgenden Beispielen beziehen sich alle Prozentangaben auf Gewichtsprozente.

*Beispiel 1*

Herstellung der Diisocyanate durch Phosgenierung der entsprechenden Diamine - Allgemeine Arbeitsvorschrift.

Zu einer Lösung von 495 g (5 Mol) Phosgen in 1500 ml 1,2-Dichlorethan wird innerhalb von 30 Minuten bei —10 bis 0°C eine Lösung bzw. Suspension von 1,12 Mol eines Diaminoarylsulfonamids in 1300 ml 1,2-Dichlorethan gegeben. Die Reaktionslösung wird anschliessend 3 Stunden unter einem konstanten Phosgenstrom am Rückfluss erhitzt und danach innerhalb von 3 Stunden Phosgenreste mit einem Stickstoffstrom ausgeblasen. Die klare Reaktionslösung wird am Rotationsverdampfer eingeengt.

Das in annähernd quantitativer Ausbeute erhaltene Diisocyanat-Rohprodukt liegt mit seinem NCO-Gehalt nahe am theoretischen Wert und kann durch Umkristallisieren oder Destillation im Ölpumpenvakuum gereinigt werden.

Dargestellte Produkte:

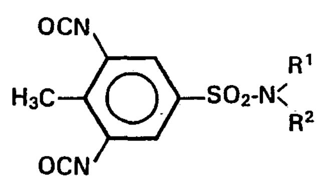

I

| | $R^1$ | $R^2$ | %NCO (theor.) | (prak.) | Fp. |
|---|---|---|---|---|---|
| I a | $n-C_4H_9$ | $n-C_4H_9$ | 23,01 | 22,9% | 58-59°C |
| I b | $CH_3$ | $n-C_{18}-H_{37}$ | 16,2 | 15,1% | 67-69°C |
| I c | $R^1+R^2=$ | $-(CH_2)_4-$ | 27,4 | 25,1% | 117-120°C |

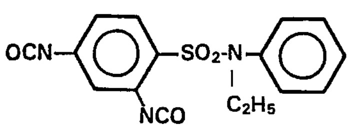

%NCO 24,5 (theor.)
23,1 (prakt.)
Fp.: 77-79°C

II

$^1$H-NMR-, IR-Spektren und Elementaranalysen stimmen mit den obigen Strukturen überein.

*Beispiel 2*

Herstellung von 1-(N,N-Di-n-butyl-sulfonamido)--2,4-diisocyanato-5-methyl-benzol durch Phosgenierung des entsprechenden Bisurethans.

Zu einer Lösung von 54,5 g (0,2 Mol) 1-Chlorsulfonyl-2,4-diisocyanato-5-methyl-benzol und 0,05 g Zinn(II)-ethylhexoat in 200 ml Ether wurden innerhalb von 1 Stunde bei Raumtemperatur 33,3 g (0,45 Mol) tertiäres Butanol getropft. Nach 4stündigem Rühren wird von wenig Feststoff abgesaugt und zu dem Filtrat innerhalb 1 Stunde eine Lösung von 54 g (0,42 Mol) Dibutylamin in 50 ml Ether zugesetzt. Das abgeschiedene Dibutylaminochlorid wird abfiltriert und das Filtrat eingeengt. Der ölige Rückstand (80 g) des Bisurethans erstarrt alsbald zu einer kristallinen Masse (Fp. = 110-115°C).

Analyse

| | | | | |
|---|---|---|---|---|
| gef.: | C 58,5 | H 8,4 | N 8,2 | S 6,2 |
| ber.: | C 58,3 | H 8,3 | N 8,2 | S 6,6 |

In eine ca. 20gew.-%ige Lösung des Bisurethan in 1,2-Dichlorethan wird bei Raumtemperatur Phosgen eingeleitet und der Reaktionsansatz allmählich auf 70°C erhitzt. Danach wird das überschüssige Phosgen mit Stickstoff ausgetrieben und das Dichlorethan abgedampft. Das Sulfonamidgruppen aufweisende Diiso-cyanat fällt als rohes Öl mit einem NCO-Gehalt von 19,8% (berechnet: 23,0%) an und erstarrt zu einem Feststoff mit einem Schmelzpunkt von 50-55°C.

*Beispiel 3*

Herstellung eines erfindungsgemässen Diisocyanats durch Einwirkung von Chlorwasserstoff auf einen dem Diisocyanat zugrundeliegenden N,N-disubstituierten Harnstoff:

a) *Herstellung des Bis-dimethylharnstoffes von 1--Methyl-4-dimethylaminosulfonyl-2,6-diisocyanat*

Zu einer Lösung von 100 ml 52%igem Dimethylamin in Wasser gibt man auf einmal 27,2 g (100 mmol) 4-Chlorsulfonyl-2,6-diisocyanatotoluol. Man lässt die exotherme Reaktion auslaufen und rührt 1 Stunde nach. Das Reaktionsgemisch wird durch Einengen auf ca. 1/3 des Ausgangsvolumens vom überschüssigen Amin befreit. Nach Absaugen und Trocknen erhält man 27,4 g = 74% des obigen Bisharnstoffsulfonamids. Der Schmelzpunkt beträgt 237 bis 238°C.

b) *4-Dimethylaminosulfonyl-2,6-diisocyanato-toluol*

7,4 g (20 mmol) des obigen Produkts werden in 100 ml Chlorbenzol gelöst. Man erhitzt die Lösung auf Rückfluss und leitet dann gleichzeitig Kohlendioxid und Chlorwasserstoff ein. Nach 20' wird die Chlorwasserstoffzufuhr unterbrochen und nach weiteren 20' Ausblasen mit Kohlendioxid wird die Reaktionslösung auf Raumtemperatur abgekühlt und das ausgefallene Dimethylaminhydrochlorid abgesaugt (86%). Nach Abdestillieren des Chlorbenzols erhält man 5,92 g Rohware (80%). Die Rohware wurde im Hochvakuum bei 200 bis 230°C Badtemperatur destilliert (0,1 Torr).

Fp.: 111-112°C; NCO ber.: 30,0%; gef.: 29,9%.

Infrarot- und Kernresonanz-Spektrum sind mit der Struktur im Einklang.

*Beispiel 4*

Vergleich der Reaktionsgeschwindigkeiten von Diisocyanatoarylsulfonamiden mit den entsprechenden aromatischen Diisocyanaten.

Zur Ermittlung der relativen Reaktionsgeschwindigkeiten wird folgender Versuch durchgeführt: Bei 23 ± 0,5°C werden 0,004 Mol eines Diisocyanates in 100 ml Toluol vorgelegt. Zum Zeitpunkt t = 0 min wird eine Lösung von 0,08 Mol n-Butanol in 100 ml Toluol zugesetzt. Dann wird die Abnahme des NCO-Gehaltes, die ein Mass für den Umsatz ist, über die Zeit t verfolgt. In der nachfolgenden Tabelle wird die Abnahme des NCO-Gehalts in Abhängigkeit von der Zeit gezeigt. Die in Beispiel 1 beschriebenen erfindungsgemässen Diisocyanate Ia und II werden mit 2,4-Diisocyanatotoluol («2,4 T») und 2,6-Diisocyanatotoluol («2,6-T») verglichen.

Tabelle 1

NCO-Abnahme in Abhängigkeit von der Zeit

| Verb./Min | 1 | 5 | 10 | 20 | 50 | 100 | 200 | 300 |
|---|---|---|---|---|---|---|---|---|
| I a | 0,152 | 0,130 | 0,120 | 0,108 | 0,076 | 0,040 | 0 | |
| II | 0,082 | 0,0469 | 0,030 | 0,004 | 0 | | | |
| 2,4-T | 0,199 | 0,172 | 0,158 | 0,139 | 0,085 | 0,085 | 0,063 | 0,048 |
| 2,6-T | 0,197 | 0,181 | 0,174 | 0,165 | 0,148 | 0,127 | 0,095 | 0,072 |

*Beispiel 5*

Nachstehend werden durch Umsetzung des erfindungsgemässen Diisocyanats Ia aus Beispiel 1 bzw. durch Umsetzung von 2,6-Diisocyanatotoluol mit äquimolaren Mengen Butandiol-1,4 Urethane hergestellt und thermogravimetrisch bzw. differentialkalorimetrisch untersucht:

Jeweils 0,028 Mol der genannten Diisocyanate werden mit 0,028 Mol Butandiol-1,4 in 80 ml Chlorbenzol 4 Stunden auf 80°C erhitzt. Das so erhaltene Polyurethan auf Basis des erfindungsgemässen Diisocyanats Ia weist einen Schmelzpunkt bzw. Schmelzbereich von 137-150°C auf. Das entsprechende Polyurethan auf Basis des vergleichsdiisocyanats weist einen Schmelzbereich von 228 bis 235°C auf.

Mit beiden Polyurethanen wurden thermogravimetrische und differentialkalorimetrische Untersuchungen durchgeführt. Zur thermogravimetrischen Messung werden die Materialien in einer handelsüblichen Thermowaage unter Stickstoff mit 20 K/min (Erhitzung um 20°C pro Minute) bis zur vollständigen Zersetzung der Probe aufgeheizt. Die thermoanalytischen Messungen wurden mit einem handelsüblichen Differential-Scanning-Kalorimeter unter Stickstoff im Temperaturbereich von 15 bis 150°C durchgeführt. Die Aufheizgeschwindigkeit betrug ebenfalls 20 K/min.

| | Ia | 2,6-T |
|---|---|---|
| Maximum in der differenzierten thermogravimetrischen Messung (Maximum der Zersetzungsgeschwindigkeit) | 363°C | 345°C |
| Glasübergangstemperatur bei der differential-kalorimetrischen Messung | 51°C | 80°C |

Die Verbindung aus dem erfindungsgemässen Diisocyanat weist eine niedrigere Glasübergangstemperatur bei gleichzeitig um ca. 20°C höherer Wärmebeständigkeit auf.

*Beispiel 6*

Nachstehend werden Elastomere, hergestellt mit dem erfindungsgemässen Diisocyanat Ia aus Beispiel 1 bzw. mit 2,4-Diisocyanatotoluol, miteinander verglichen. Zunächst wurden die nachstehend beschriebenen Präpolymere A)-D) hergestellt.

A) 2000 g eines aus Adipinsäure und Ethylenglykol erhaltenen Polyesters (MG 2000; OH-Zahl = 56) werden mit 739 g Diisocyanat Ia auf 70-80°C erwärmt und bis zu einem NCO-Gehalt von 2,9-3,0 Gew.-% bei dieser Temperatur gehalten.

B) NCO-Präpolymer entsprechend A), jedoch hergestellt unter Verwendung von 348 g 2,4-Diisocyanatotoluol. Der NCO-Gehalt des NCO-Präpolymeren liegt bei 3,5 Gew.-%.

C) Der im Beispiel A) genannte Polyester wird durch einen Polyether ersetzt, der durch Addition von Propylenoxid an Propylenglykol erhalten wurde (MG 2000; OH-Zahl = 56).

D) Der in Beispiel B) genannte Polyester wird durch den in Beispiel C) genannten Polyether ersetzt.

300 g der NCO-Präpolymeren A)-D) werden bei 80°C kurz im Wasserstrahlvakuum entgast und innerhalb von 30 Sekunden mit geschmolzenem 3,5-Diamino-4-chlorbenzoesäureisobutylester innig vermischt. Die Menge des Diamins wird so bemessen, dass das molare Verhältnis von NCO zu $NH_2$ 1,1 : 1 beträgt. Der Reaktionsansatz wird in eine auf 100°C erwärmte Form gegossen und nach der Entformung noch 24 Stunden bei 110°C getempert.

In der nachfolgenden Tabelle 2 werden die mechanischen Eigenschaften der so hergestellten Elastomeren mitgeteilt. Es kann im wesentlichen die Schlussfolgerung gezogen werden, dass die mit dem erfindungsgemässen Diisocyanat hergestellten Präpolymere eine erhöhte Reaktivität verglichen mit dem Diisocyanat des Standes der Technik aufweist. Die mit dem erfindungsgemässen Diisocyanat hergestellten Elastomeren stellen ausserdem weichere Produkte bei gleichbleibender Stosselastizität dar.

Tabelle 2

| Präpolymer | Giesszeit (min) | Abhebzeit (min) | Zugspannung ($kp/cm^2$) 100% DIN 53504 | 300% | Reissdehnung (%) DIN 53504 | Weiterreisswiderstand (kp/cm) DIN 53515 | Shore A DIN 53505 | Shore D | Stosselastizität (%) DIN 53512 |
|---|---|---|---|---|---|---|---|---|---|
| A | 0,5 | 1 | 4,19 | 5,57 | 531 | 30,9 | 78 | 24 | 31 |
| B | 10 | 12 | 6,09 | 10,0 | 701 | 64,8 | 85 | 30 | 36 |
| C | 8 | 10 | — | — | 386 | 11,4 | 75 | 16 | 38 |
| D | 200 | 45 | 4,40 | 4,77 | 422 | 19,0 | 78 | 19 | 42 |

**Patentansprüche**

1. Diisocyanate der allgemeinen Formel

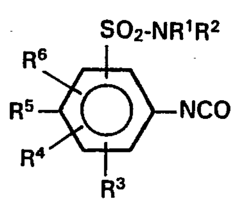

in der

$R^1$ und $R^2$ für gleiche oder verschiedene Reste stehen und Alkylreste mit 1 bis 18 Kohlenstoffatomen, Arylreste mit 6 bis 10 Kohlenstoffatomen, Cycloalkylreste mit 3 bis 7 Kohlenstoffatomen, Aralkylreste mit 7 bis 15 Kohlenstoffatomen oder Alkarylreste mit 7 bis 15 Kohlenstoffatomen bedeuten, oder zusammen mit dem Stickstoffatom einen gegebenenfalls weitere Heteroatome enthaltenden heterocyclischen Ring mit 3 bis 10 Ringgliedern bilden,

$R^3$, $R^4$ und $R^5$ für gleiche oder verschiedene Reste stehen und Wasserstoff, Alkylreste mit 1 bis 6 Kohlenstoffatomen, Arylreste mit 6 bis 10 Kohlenstoffatomen, Cycloalkylreste mit 3 bis 7 Kohlenstoffatomen, Aralkylreste mit 7 bis 15 Kohlenstoffatomen, Alkarylreste mit 7 bis 15 Kohlenstoffatomen oder Halogenatome bedeuten,

$R^6$ für eine Isocyanatgruppe oder einen Rest der Formel

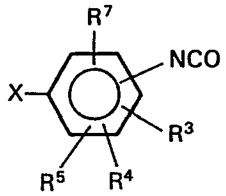

steht, wobei

$R^7$ für Wasserstoff oder einen Rest der Formel

$$-SO_2\ NR^1R^2$$

steht und

X für eine Methylen- oder eine Dimethylmethylen-Gruppe steht.

2. Diisocyanate der in Anspruch 1 genannten Formel, für welche

$R^1$ für einen Alkylrest mit 1 bis 6 Kohlenstoffatomen steht,

$R^2$ für einen Alkylrest mit 1 bis 18 Kohlenstoffatomen steht,

$R^3$ und $R^4$ für Wasserstoff stehen,

$R^5$ für Wasserstoff, einen Alkylrest mit 1 bis 4 Kohlenstoffatomen oder Chlor steht und

$R^6$ für eine Isocyanatgruppe steht.

3. Verfahren zur Herstellung von Diisocyanaten gemäss Anspruch 1 und 2, dadurch gekennzeichnet, dass man die den Diisocyanaten zugrundeliegenden Diamine in an sich bekannter Weise phosgeniert.

4. Verfahren zur Herstellung von Diisocyanaten gemäss Anspruch 1 und 2, dadurch gekennzeichnet, dass man die den Diisocyanaten entsprechenden Bisurethane tertiärer Alkohole mit Phosgen behandelt.

5. Verfahren zur Herstellung von Diisocyanaten gemäss Anspruch 1 und 2, dadurch gekennzeichnet, dass man den Diisocyanaten entsprechende, 2 Struktureinheiten der Formel

$$-NH-\overset{\overset{\displaystyle O}{\|}}{C}-NR^1R^2$$

aufweisende Bisharnstoffe unter Abspaltung von sekundären Aminen der Formel

$$HNR^1R^2$$

und gleichzeitiger Bildung der entsprechenden Ammoniumsalze der Formel

$$[H_2NR^1R^2]^+\ Cl^{(-)}$$

mit Chlorwasserstoff behandelt.

6. Verwendung der Diisocyanate gemäss Anspruch 1 und 2 als Aufbaukomponente bei der Herstellung von Polyurethankunststoffen nach dem Isocyanat-Polyadditionsverfahren.

**Claims**

1. Diisocyanates of the general formula

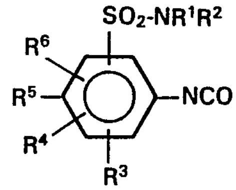

in which

$R^1$ and $R^2$ represent identical of different radicals and denote alkyl radicals with 1 to 18 carbon atoms, aryl radicals with 6 to 10 carbon atoms, cycloalkyl radicals with 3 to 7 carbon atoms, aralkyl radicals with 7 to 15 carbon atoms or alkaryl radicals with 7 to 15 carbon atoms, or together with the nitrogen atom form a heterocyclic ring which has 3 to 10 ring members and optionally contains further heteroatoms,

$R^3$, $R^4$ and $R^5$ represent identical or different radicals and denote hydrogen, alkyl radicals with 1 to 6 carbon atoms, aryl radicals with 6 to 10 carbon atoms, cycloalkyl radicals with 3 to 7 carbon atoms, aralkyl radicals with 7 to 15 carbon atoms, alkaryl radicals with 7 to 15 carbon atoms or halogen atoms,

$R^6$ represents an isocyanate group or a radical of the formula

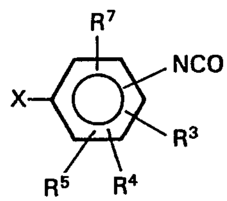

wherein

$R^7$ represents hydrogen or a radical of the formula

$$-SO_2\ NR^1R^2$$

and

X represents a methylene or a dimethylmethylene group.

2. Diisocyanates of the formula mentioned in claim 1, for which

$R^1$ represents an alkyl radical with 1 to 6 carbon atoms,

$R^2$ represents an alkyl radical with 1 to 18 carbon atoms,

$R^3$ and $R^4$ represent hydrogen,

$R^5$ represents hydrogen, an alkyl radical with 1 to 4 carbon atoms or chlorine, and

$R^6$ represents an isocyanate group.

3. Process for the production of diisocyanates according to claim 1 and 2, characterised in that the diamines on which the diisocyanates are based are phosgenated in a manner known per se.

4. Process for the production of diisocyanates according to claim 1 and 2, characterised in that the bis-urethanes of tertiary alcohols corresponding to the diisocyanates are treated with phosgene.

5. Process for the production of diisocyanates according to claim 1 and 2, characterised in that bis-ureas containing two structural units of the formula

$$-NH-\overset{\overset{O}{\|}}{C}-NR^1R^2$$

and corresponding to the diisocyanates, are treated with hydrogen chloride with elimination of secondary amines of the formula

$$HNR^1R^2$$

and simultaneous formation of the corresponding ammonium salts of the formula

$$[H_2NR^1R^2]^+ \; Cl^{(-)}$$

6. Use of the diisocyanates according to claim 1 and 2 as a starting component in the production of polyurethane plastics by the isocyanate-polyaddition process.

**Revendications**

1. Diisocyanates de formule générale

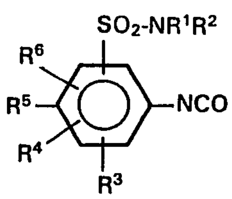

dans laquelle

$R^1$ et $R^2$ sont des restes égaux ou différents et désignent des restes alkyle ayant 1 à 18 atomes de carbone, des restes aryle ayant 6 à 10 atomes de carbone, des restes cycloalkyle ayant 3 à 7 atomes de carbone, des restes aralkyle ayant 7 à 15 atomes de carbone ou des restes alkaryle ayant 7 à 15 atomes de carbone, ou forment conjointement avec l'atome d'azote un noyau hétérocyclique de 3 à 10 chaînons, contenant éventuellement d'autres hétéroatomes,

$R^3$, $R^4$ et $R^5$ sont des restes égaux ou différents et représentent de l'hydrogène, des restes alkyle ayant 1 à 6 atomes de carbone, des restes aryle ayant 6 à 10 atomes de carbone, des restes cycloalkyle ayant 3 à 7 atomes de carbone, des restes aralkyle ayant 7 à 15 atomes de carbone, des restes alkaryle ayant 7 à 15 atomes de carbone ou des atomes d'halogènes,

$R^6$ désigne un groupe isocyanate ou un reste de formule

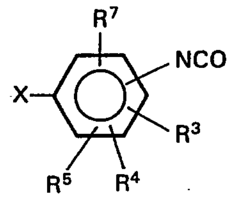

dans laquelle

$R^7$ représente l'hydrogène ou un reste de formule

$$-SO_2\,NR^1R^2$$

et

X est un groupe méthylène ou un groupe diméthylméthylène.

2. Diisocyanates de formule mentionnée dans la revendication 1, dans lesquels

$R^1$ représente un reste alkyle ayant 1 à 6 atomes de carbone,

$R^2$ représente un reste alkyle ayant 1 à 18 atomes de carbone,

$R^3$ et $R^4$ sont de l'hydrogène,

$R^5$ est de l'hydrogène, un reste alkyle ayant 1 à 4 atomes de carbone ou du chlore et

$R^6$ représente un groupe isocyanate.

3. Procédé de production de diisocyanates suivant les revendications 1 et 2, caractérisé en ce qu'on phosgène d'une manière connue les diamines qui sont à la base des diisocyanates.

4. Procédé de production de diisocyanates suivant les revendications 1 et 2, caractérisé en ce qu'on traite au phosgène les bis-uréthannes d'alcools tertiaires correspondant aux diisocyanates.

5. Procédé de production de diisocyanates suivant les revendications 1 et 2, caractérisé en ce qu'on traite avec du chlorure d'hydrogène des bis-urées correspondant aux diisocyanates, présentant 2 motifs structuraux de formule

$$-NH-\overset{\overset{O}{\|}}{C}-NR^1R^2$$

avec élimination d'amines secondaires de formule

$$HNR^1R^2$$

et formation simultané des sels d'ammonium correspondant de formule

$$[H_2NR^1R^2]^+ \; Cl^{(-)}$$

6. Utilisation des diisocyanates suivant les revendications 1 et 2 comme composant structural dans la production de matières plastiques du type polyuréthanne par le procédé de polyaddition d'isocyanate.